# FASCICULE DE BREVET EUROPEEN

(11) **EP 0 646 353 B1**
(45) Date de publication et mention de la délivrance du brevet: **26.11.1997**
(21) Numéro de dépôt: 94420247.2
(22) Date de dépôt: 15.09.1994
(51) Int. Cl.: A61B 17/064

(54) **Agrafe d'ostéosynthèse multi-branches à compression dynamique auto-rétentive**
Mehrzweigige Osteosyntheseklammer, die eine dynamische selbstretentionierte Komprimierung besitzt
Multi-branched ostheosynthesis staple having a dynamic autoretentive compression

(30) Priorité: 21.09.1993 FR 9311429
(43) Date de publication de la demande: 05.04.1995
(73) Titulaire: Mai, Christian, F-69006 Lyon (FR)
(72) Inventeur: Mai, Christian, F-69006 Lyon (FR)
(74) Mandataire: Laurent, Michel

(56) Documents cités:
- EP-A- 0 145 166
- EP-A- 0 488 906
- US-A- 4 485 816

## Description

L'invention concerne un nouveau type d'agrafe d'ostéosynthèse multi-branches présentant des caractéristiques de compression dynamique, d'auto-rétention et de stabilité mécanique.

Par "compression dynamique", on entend la faculté que présentent certaines agrafes de générer une force de compression résultante entre les points au niveau desquels elles sont implantées et, plus particulièrement de part et d'autre d'un foyer de fracture osseux.

Les agrafes chirurgicales utilisées pour la réduction des fractures et la fixation des os et des tissus mous, doivent posséder plusieurs caractéristiques essentielles. Tout d'abord, elles doivent développer un effet de compression constant dans le temps. Elles doivent être bien ancrées de telle sorte à éviter leur décrochement après implantation, décrochement généralement inhérent aux mouvements de l'articulation ou simplement de l'os sur lequel elles sont implantées. En outre, la stabilité de la zone de fracture par une bonne immobilisation constitue une condition importante pour aboutir à une consolidation osseuse. Enfin, le mode d'implantation ou d'enlèvement des agrafes doit être simple, facile à mettre en oeuvre, et générant un minimum de traumatismes osseux.

Parmi les différents types d'agrafes connus à ce jour, il en est un dans lequel lesdites agrafes sont réalisées en un matériau martensitique, par exemple en un alliage du type nickel/titane ou titane/niobium, et auxquelles on confère un phénomène dit de mémoire de forme, susceptible d'induire un rapprochement de leurs extrémités lorsque l'agrafe se situe à une température supérieure à la température de transformation austénitique dudit matériau qui la compose. Ce phénomène de mémoire de forme est du à la transformation martensitique thermo-élastique reversible. Ce phénomène aujourd'hui bien connu, consiste à donner à un matériau une forme définie que l'on traite à une température supérieure à la température de transformation austénitique Aₛ du matériau, puis à lui donner une autre forme également définie à une température inférieure à la température martensitique Mₛ dudit matériau, et à répéter un certain nombre de fois cette opération, en fonction de la nature de l'alliage utilisé, afin de donner à ce matériau sa mémoire de forme définitive. La température martensitique est inférieure à la température austénitique.

Si certes ces agrafes permettent de générer une compression dynamique au niveau de l'extrémité de leurs branches, celle-ci s'avère généralement insuffisante pour la totalité de la fracture au niveau de laquelle elles sont implantées et même parfois rédhibitoire, car cette compression est disymétrique. En effet, elle a tendance à rapprocher la partie profonde de la zone de la fracture et à écarter la partie superficielle de cette même zone.

Afin de pallier ces inconvénients, on a proposé dans le document EP-A-0 488 906 du Demandeur, une agrafe d'ostéosynthèse réalisée en un matériau martensitique dans lequel, le passage de la température de la température martensitique à la température austénitique, induit un raccourcissement de la longueur de la base de l'agrafe, au moins partiellement, et, corolairement, le rapprochement des extrémités desdites branches constitutives de l'agrafe. Outre la génèse par ce type d'agrafe d'une compression dynamique constante dans le temps et homogène, elle permet également une compression tant au niveau de la partie superficielle de l'os que dans sa partie profonde, compte tenu du fait que les extrémités des branches qui la constituent sont également éduquées pour se rapprocher l'une de l'autre.

Cependant, toutes les agrafes à deux branches ne confèrent pas une stabilité mécanique au foyer de la fracture osseuse. De fait, il est souvent nécessaire de positionner plusieurs agrafes pour aboutir à cette stabilité, induisant plusieurs opérations consécutives, conduisant en outre à une stabilité imparfaite.

Par ailleurs, ce type d'agrafe est de réalisation délicate, compte tenu de sa forme particulière, notamment du ménagement au niveau de sa base d'une portion de section réduite et donc générant une éducation difficile à induire.

L'objet de l'invention est de proposer une agrafe d'ostéosynthèse multi-branches du type en question permettant en une seule opération d'assurer par sa forme et sa capacité d'adaptation à la forme de l'os à réparer, la stabilité mécanique des deux ou plusieurs éléments de l'os de la zone de fracture.

Cette agrafe d'ostéosynthèse réalisée en un alliage martensitique thermo-élastique, dont les températures de transformation martensitique Mₛ et austénitique Aₛ peuvent varier, en fonction des applications, entre - 20 ° C à 70 ° C, comporte des branches latérales destinées à être insérées de part et d'autre du foyer de la fracture d'un os à réparer, lesdites branches étant raccordées entre elles par au moins une portion de raccordement, les branches et la portion de raccordement étant éduquées pour respectivement se recourber sensiblement vers le centre de l'agrafe et pour se raccourcir sous l'effet de la température, lorsque celle-ci dépasse la température de transformation austénitique Aₛ du matériau qui la compose.

Elle se caractérise en ce qu'elle est constituée d'un fil unitaire et monobloc, avec lequel au moins l'une des branches qui la constituent est réalisée par repliage au moins partiel dudit fil sur lui-même. Les deux extrémités libres du fil peuvent constituer chacune une autre branche, lesdites branches étant destinées à venir s'insérer dans ou au contact de l'os au niveau de l'un des bords de la fracture.

Selon l'invention, il est possible d'obtenir un certain nombre de branches supplémentaires constituées par le repliage partiel du fil sur lui-même.

Selon l'invention, la portion de raccordement de l'agrafe éduquée de telle sorte à se racourcir sous l'effet de la température, adoptant une forme au moins partiellement recourbée lors du passage à une température supérieure à la température de transformation austénitique du matériau martensitique qui la constitue, est également constituée par le repliage partiel du fil constitutif de l'agrafe, dans le prolongement de la ou des branches latérales ainsi constituées.

Selon une autre caractéristique de l'invention, la ou les branches latérales constituées par repliage partiel du fil constitutif sur lui-même, sont susceptibles d'adopter une forme recourbée sous l'effet de la température. De la sorte, on améliore les capacités d'auto-rétention de l'agrafe.

Selon une autre caractéristique de l'invention, le raccourcissement de la portion de raccordement de l'agrafe sous l'effet de la température est obtenu par rabat partiel en direction du milieu de ladite agrafe des éléments de la portion constitués par le fil seul et non par repliage partiel de celui-ci sur lui-même.

Selon une autre caractéristique de l'invention, le raccourcissement de la portion de raccordement de l'agrafe sous l'effet de la température est obtenu sous l'effet combiné de l'adoption d'une forme recourbée de la zone de ladite portion constituée par repliage partiel du fil constitutif, et par le rabat en direction du milieu de l'agrafe des éléments de la portion constitués par le fil seul.

Selon une première forme de réalisation de l'invention, l'agrafe en forme de Y comporte trois branches, dont l'une est constituée par repliage partiel du fil constitutif de l'agrafe sur lui-même, les deux autres branches étant susceptibles de se rapprocher ou de s'écarter l'une de l'autre lors du passage à une température supérieure à la température de transformation austénitique du matériau.

Dans une autre forme de réalisation dans laquelle l'agrafe comporte également trois branches, celles-ci sont réparties de telle sorte à conférer à l'agrafe une forme en T, à savoir une branche principale constituée par repliage partiel et les deux autres branches situées à l'extrémité de la barre supérieure du T étant constituées par les deux extrémités libres.

Dans une autre forme de réalisation dans laquelle l'agrafe comporte également trois branches, celles-ci sont réparties de telle sorte à conférer à l'agrafe une forme en V, dans laquelle l'une des branches est constituée par repliage partiel sur lui-même du fil constitutif de l'agrafe, et dans laquelle les deux autres branches sont susceptibles de se rapprocher ou de s'écarter l'une de l'autre lors du passage à une température supérieure à la température de transformation austénitique du matériau.

Dans une autre forme de réalisation dans laquelle l'agrafe comporte également trois branches, celles-ci sont réparties de telle sorte à conférer à l'agrafe une forme en tabouret, dans laquelle chacune des branches est constituée par repliage partiel du fil constitutif de l'agrafe sur lui-même.

Dans une autre forme de réalisation, l'agrafe comporte quatre branches, réparties de telle sorte à conférer à l'agrafe une forme en double Y, la barre centrale étant commune aux deux Y, les deux branches de l'un d'entre eux étant constituées par repliage partiel sur lui-même du fil, et les deux branches de l'autre étant constituées par les deux extrémités libres dudit fil, les deux branches de chacun des deux jeux étant également susceptibles de se rapprocher ou de s'écarter, en fonction de l'éducation en mémoire de forme qui leur est donnée, lors du passage de la température à une température supérieure à la température de transformation austénitique du matériau.

Dans une autre forme de réalisation à quatre branches, l'agrafe présente une forme en double T, reliés l'un à l'autre par leur barre principale, deux des branches étant réalisées par repliage partiel sur lui-même du fil, les deux autres étant constituées par les deux extrémités libres dudit fil.

Dans une autre forme de réalisation également à quatre branches, l'agrafe présente une forme en tabouret, chacune des branches dudit tabouret étant constituée par le repliage partiel du fil sur lui-même, les deux extrémités libres du fil venant se rejoindre au niveau de l'une des branches. Selon l'éducation donnée à l'agrafe, les différentes portions de raccordement des branches entre elles adoptent une forme ondulée lors du passage de la température à une température supérieure à la température de transformation austénitique du matérieu, ou seules deux portions de raccordement opposées subissent un tel raccourcissement.

La manière dont l'invention peut être réalisée et les avantages qui en découlent, ressortiront mieux des exemples de réalisation qui suivent donnés à titre indicatif mais non limitatif, à l'appui des figures annexées.

Les figures 1 et 2a, 2b et 2c représentent une première forme de réalisation de l'invention à trois branches en forme de Y, respectivement à une température inférieure à la température de transformation martensitique et supérieure à la température de transformation austénitique du matériau qui la compose.

Les figures 3 et 4a, 4b sont des représentations schématiques de l'agrafe en forme de Y des figures 1 et 2 en place au niveau d'une zone de fracture osseuse.

Les figures 5 et 6 sont des représentations similaires aux figures 1 et 2, d'une agrafe à trois branches en forme de T.

La figure 7 est une représentation schématique de la mise en place de l'agrafe des figures 5 et 6 au niveau d'un foyer de fracture osseuse.

Les figures 8 et 9a, 9b et 9c sont des représentations similaires aux figures 1 et 2, d'une agrafe à quatre branches en forme de double Y.

Les figures 10 et 11 sont des représentations similaires aux figures 1 et 2, d'une agrafe à quatre branches en forme de double T.

Les figures 12 et 13 sont des figures analogues à celles des figures 10 et 11 d'une agrafe également en forme de double T, selon ne autre forme de réalisation de l'invention.

La figure 14 est une représentation schématique de la mise en place de l'agrafe des figures 12 et 13 au niveau d'un foyer de fracture osseuse.

Les figures 15 et 16 sont des représentations similaires aux figures 1 et 2, d'une agrafe à trois branches en forme de V.

Les figures 17 et 18 sont des représentations schématiques similaires à celles des figures 1 et 2 d'une agrafe à trois branches en forme de tabouret.

Les figures 19, 20 et 21 sont des représentations schématiques similaires aux figures 1 et 2 d'une agrafe à quatre branches en forme de tabouret, la figure 21 étant une variante de la figure 20.

Les agrafes d'ostéosynthèse selon l'invention sont réalisées en un matériau martensitique répondant aux propriétés de bio-compatibilité requises. Typiquement, ce matériau martensitique est constitué par un alliage en nickel/titane ou en un alliage à base de cuivre, d'aluminium ou de zinc.

L'agrafe d'ostéosynthèse (1) selon l'invention comprend fondamentalement des branches latérales (2, 3, 4), reliées entre elles par une base ou portion de raccordement (5, 6, 7, 8), l'ensemble de ces différents éléments étant constitué au moyen d'un seul fil monobloc de section circulaire ou polygonale (carré, rectangulaire), etc., réalisé dans ledit alliage.

A la température en dessous de Mₛ, les branches latérales (2, 3 et 4) décrites plus en détail ci-après, sont rectilignes et dirigées sensi-blement perpendiculairement par rapport à la portion de raccordement.

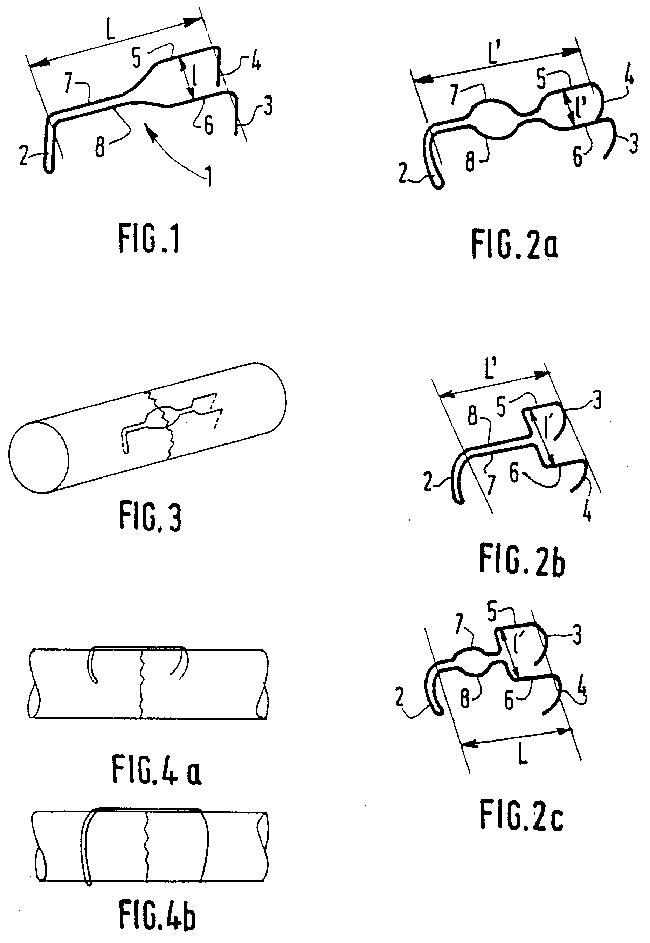

Dans une première forme de réalisation décrite notamment en liaison avec les figures 1 à 4b, l'agrafe comporte trois branches latérales (2,3 et 4) et la portion de raccordement forme un Y, dont la base (7, 8) est constituée par repliage partiel du fil constitutif de l'agrafe sur lui-même, et qui se poursuit par deux branches divergentes (5, 6), constituées par le fil de manière unitaire, et dont les deux extrémités libres formes deux des branches latérales (3, 4). L'agrafe est ainsi constituée d'un seul fil monobloc, replié sur lui-même, la branche extrême (2) étant constituée par le repliage partiel du fil sur lui-même, et se poursuivant par la base du Y. Le fil est éduqué de telle sorte que lorsque la température est inférieure à la température martensitique Mₛ du matériau qui le compose, les branches latérales (2, 3, 4) sont rectilignes, ainsi que les éléments (5, 6, 7, 8) constituant la portion de raccordement dudit Y et, lorsque la température est supérieure à la température de transformation austénitique Aₛ dudit matériau, les branches latérales adoptent une position recourbée dirigée vers l'intérieur de l'agrafe, tel que représenté sur la figure 2a, et en outre, la portion de raccordement (7,8) adopte également un profil ondulé ou courbe, par exemple situé dans le plan de la dite portion, de telle sorte à obtenir un raccourcissement de la longueur initiale L de ladite agrafe selon une nouvelle valeur L' (L' < L) (voir figure 2a).

Cette mémoire de forme peut être acquise par les différents éléments, à savoir les branches latérales (2, 3, 4) et la portion de raccordement (7, 8) en leur donnant une forme particulière à une température supérieure à la température de transformation austénitique Aₛ du matériau qui les compose, puis en leur donnant une autre forme et notamment une forme rectiligne à une température inférieure à la température de transformation martensitique Mₛ dudit matériau. En répétant un certain nombre de fois cette transformation mécanique, une mémoire de forme rectiligne est acquise respectivement pour la portion de raccordement et pour les branches latérales à une température inférieure à la température de transformation martensitique et une mémoire de forme ondulée ou courbée pour la portion de raccordement (7, 8), avec les branches latérales (2, 3, 4) adoptant une forme recourbée pour une température supérieure au seuil austénitique, sont obtenues.

Dans une variante de l'invention, l'écartement l entre les deux branches supérieures (5, 6) constitutives du Y de la portion de raccordement, et partant entre les branches latérales (2, 3), peut être soit réduit, soit augmenté lorsque la température dépasse le seuil austénitique Aₛ du matériau, et ce en fonction de l'effet désiré.

De la sorte, il résulte lors de la mise en place de l'agrafe au niveau du patient, induisant donc un passage de la température à une température supérieure à la température de transformation austénitique Aₛ du matériau, un raccourcissement de la portion de raccordement (7, 8) de l'agrafe (1), et partant un effet de compression dynamique, et parallèlement un recourbement des branches latérales (2, 3, 4) qui la constituent, pouvant également s'accompagner soit d'un écartement des branches (3,4), soit au contraire un rapprochement de celles-ci.

La mise en place de cette agrafe au niveau de la fracture s'effectue de la manière suivante. La dite agrafe est portée à une température inférieure à la température de transformation martensitique Mₛ du matériau qui la compose, la portion de raccordement (7,8) et les branches latérales étant de fait rectilignes, ces dernières étant même sensiblement perpendiculaires à ladite portion. L'agrafe est alors implantée de part et d'autre du foyer de la fracture, et ce par compaction, des pré-orifices ayant avantageusement été réalisés préalablement par le chirurgien. Lorsque la température est supérieure au seuil austénitique, l'agrafe se déforme et adopte une forme définie par sa mémoire de forme. De la sorte, on aboutit à un double effet de compression, respectivement au niveau de l'os cortical et de l'os spongieux, ainsi qu'à un effet d'auto-rétention, inhérent au recourbement des branches latérales (2, 3, 4).

On a représenté dans les figures 3 et 4, l'agrafe en place au niveau du foyer d'une fracture osseuse, dont on a matérialisé par une ligne brisée la zone de fracture. Selon un premier mode de mise en place (figure 3 et 4a), seule l'une des parois corticales de l'os est percée pour permettre le positionnement de l'agrafe. Selon un autre mode de mise en place (figure 4b), les deux parois corticales opposées de l'os sont percées, de telle sorte à permettre la traversée des branches latérales (2, 3, 4) de part en part de l'os, et partant, d'assurer une meilleure compression des deux éléments de l'os au contact l'un de l'autre, et une stabilité accrue.

Dans une variante de l'agrafe précédente, représentée sur la figure 2b, le raccourcissement de la portion de raccordement est obtenu par rabat des branches supérieures (5, 6) de ladite portion en direction de la branche latérale (2), obtenu également par mémoire de forme à une température supérieure au seuil austénitique.

Dans une variante des deux formes précédentes représentée sur la figure 2c, le raccourcissement de la portion de raccordement est obtenu par une combinaison des deux effets précédents, à savoir, à la fois par l'adoption par la base (7, 8) de ladite portion d'un profil courbe, et par le repliage ou rabat en direction de la branche latérale (2) des branches supérieures (5, 6) de cette portion.

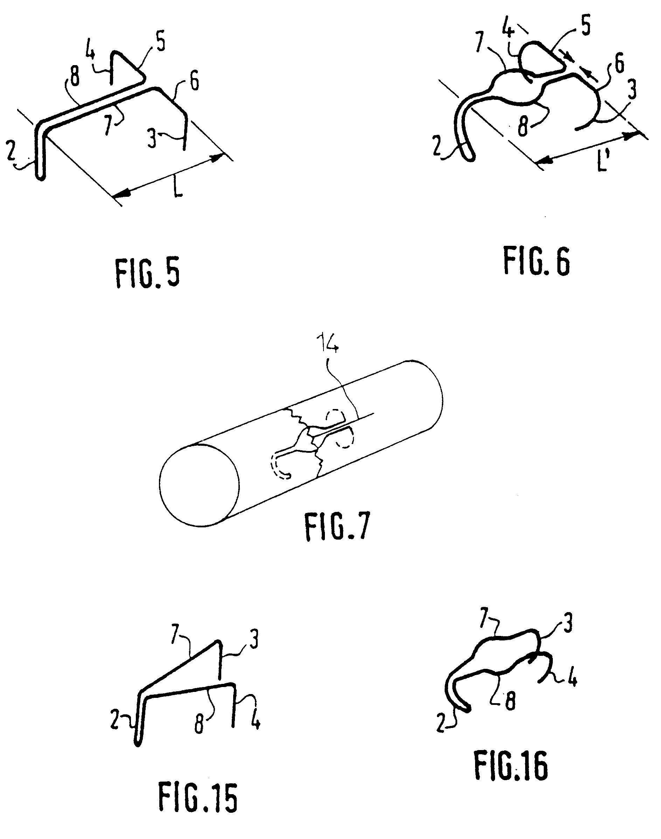

Dans une autre forme de réalisation de l'invention représentée sur les figures 5 et 6, l'agrafe comporte toujours trois branches latérales (2, 3, 4), mais la portion de raccordement entre ces branches présente une forme en T. Comme dans le cas précédent, l'une des branches (2) est constituée par le repliage partiel sur lui-même du fil qui la constitue, et la portion de raccordement (7, 8) est susceptible de se raccourcir par adoption d'un profil ondulé ou courbe lors du passage de la température au dessus du seuil austénitique. En outre, à une telle température, les deux branches supérieures (5, 6) constitutives de la portion de raccordement, et partant les deux branches latérales (3, 4) qui les prolongent, sont avantageusement éduquées pour se rapporcher l'une de l'autre, tel que matérialisé par les deux flèches sur la figure 6. En outre, les deux branches latérales (3, 4) se recourbent en direction l'une de l'autre à une telle température. De la sorte, et ainsi que cela a été représenté sur la figure 7, une telle agrafe est adaptée pour assurer d'une part, une compression au niveau d'un foyer de fracture osseuse, mais également un second effet de compression, au niveau d'une zone de fêlure (14) contigue à la fracture proprement dite, et ce en assurant un serrage effectif inhérent au rapprochement des deux branches supérieures (5, 6) de la portion de raccordement.

Dans une variante de la forme de réalisation précédente, les deux branches supérieures (5, 6) demeurent sensiblement parallèles et les branches latérales (3, 4) qui les prolongent sont susceptibles d'être introduites dans l'os à consolider, soit au niveau de deux orifices préalablement réalisés par le praticien, et ce de manière plus ou moins rapproché, soit au niveau d'un même orifice, en fonction de la dimension de l'os et de la pathologie rencontrée. Le passage de la température au dessus de la température de tranformation austénitique du matériau induit alors :
- le raccourcissement de l'agrafe, de par l'adoption par la portion de raccordement d'un profil ondulé ou en forme de boucle, et partant générant un effet de compression,
- le rapprochement des extrémités des branches latérales (3, 4), ou leur éloignement, et ce soit dans le plan général de l'agrafe, soit dans un plan perpendiculaire à ladite agrafe, soit encore, le déplacement desdites extrémités selon une même direction, et ce, en direction de la branche (2), ou au contraire dans la direction opposée à cette branche (2).

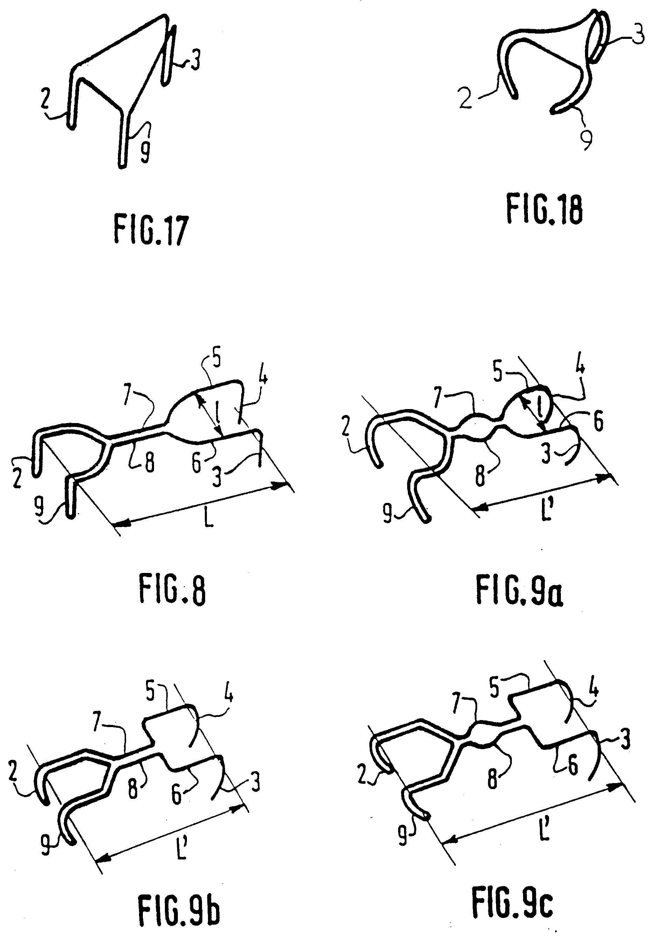

Dans une autre forme de réalisation représentée dans les figures 8 et 9, l'agrafe présente quatre branches latérales (2, 3, 4, 9) selon une forme générale de deux Y bout à bout. Deux des branches latérales (2, 9) sont constituées par repliage partiel du fil sur lui-même, et les deux autres sont constituées par les deux extrémités libres dudit fil. Ainsi qu'on peut l'observer, la portion de raccordement est constituée d'une part, par le prolongement des branches latérales (2, 9), et donc par repliage partiel sur lui-même du fil constitutif de l'agrafe, et d'autre part, par les branches supérieures (5, 6) divergeant à partir de la zone centrale de l'agrafe, et dont les extrémités libres constituent les deux branches latérales (3, 4). Comme dans le cas précédent, la portion de raccordement "double" (7, 8) est susceptible de subir une déformation par mémoire de forme selon une double ondulation ou courbure, avantageusement dans le plan de ladite portion, se matérialisant par un raccourcissement de l'agrafe (1). En outre, ce raccourcissement peut également être obtenu par rabat ou repliage partiel des branches supérieures (5, 6) de la portion de raccordement en direction des branches latérales (2, 9) (voir figure 9b), ou encore par combinaison des deux effets précédents (voir figure 9c).

Parallèlement, les deux branches latérales, respectivement (3, 4) et (2, 9) constitutives des deux jeux de branches du double Y, sont susceptibles de s'écarter ou au contraire de se rapprocher l'une de l'autre lors du passage de la température au dessus du seuil de transformation austénitique du matériau, voire l'un des jeux s'écarter et l'autre se rapprocher en fonction de l'effet désiré. Cet effet est obtenu par éducation, à l'instar des effets précédemment décrits.

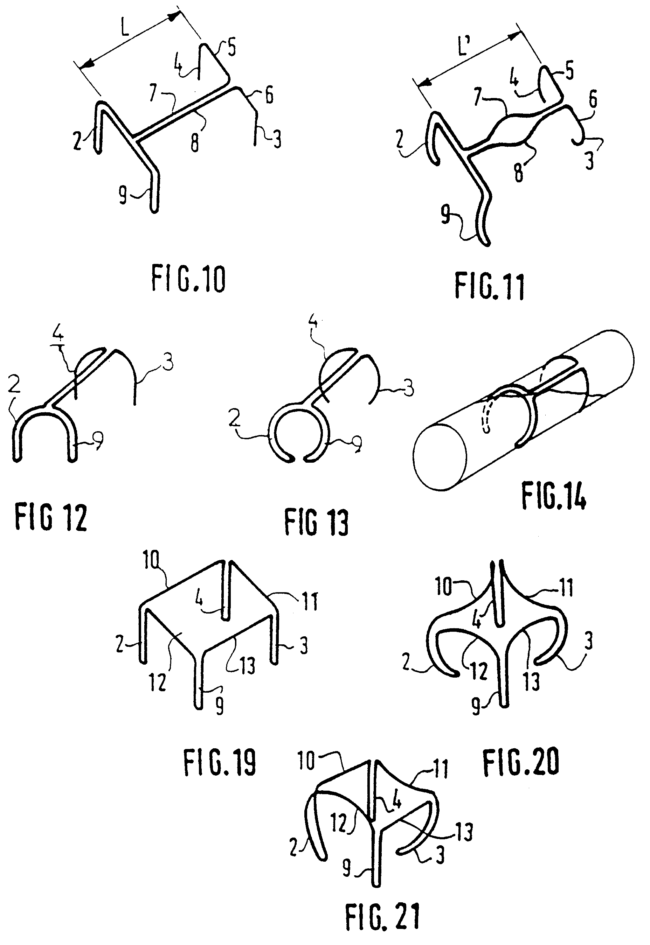

Dans une forme de réalisation représentée sur les figures 10 et 11, l'agrafe comporte quatre branches latérales (2, 3, 4, 9), raccordées entre elles par un portion de raccordement en forme de double T, comprenant respectivement deux branches latérales (2, 9) réalisées par repliage partiel sur lui-même du fil constitutif de l'agrafe, et deux branches latérales (3, 4) constituées par les deux extrémités libres dudit fil. A une température supérieure à la température de transformation austéniti-que Aₛ du matériau, on observe sur la figure 11, l'adoption par la portion de raccordement "double" (7, 8) d'un profil ondulé ou courbe, propre à induire une diminution de la distance séparant les deux jeux de deux branches latérales, et partant à assurer la compression dynamique au niveau de la fracture. Par ailleurs, lesdites branches latérales de chacun des deux jeux, respectivement (2, 9) et (3, 4) sont éduquées de telle sorte qu'à une telle température, elles se recourbent en direction du jeu de branches latérales opposé.

Dans une variante de la forme de réalisation précédente, représentée sur les figures 12 et 13, les branches latérales (2, 3, 4, 9) sont éduquées pour se présenter sous une forme légèrement cintrée à une température inférieure à la température de transformation martensitique Mₛ du matériau, et pour adopter une forme beaucoup plus cintrée (figure 13) à une température supérieure au seuil austénitique. Ce cintrage s'effectue sensiblement dans le plan intégrant les deux jeux respectifs (2, 9) et (3, 4) de branches latérales. Cette forme de réalisation de l'invention s'avère d'application avantageuse pour la réduction de fracture ou de fêlure d'os de petite taille. En effet, et tel qu'on l'a schématiquement représenté sur la figure 14, les branches latérales ne pénètrent plus dans l'os, mais l'entourent, et assurent un serrage et une compression dynamique propre à permettre sa "réparation", les dimensions de l'agrafe, et notamment le degré de cintrage des branches latérales étant choisis de telle sorte à assurer le degré de serrage voulu.

Dans une autre forme de réalisation de l'invention, dans laquelle l'agrafe comporte trois branches latérales, représentée sur les figures 15 et 16, la portion de raccordement est en forme de V. Comme dans les exemples précédents, ladite portion (7, 8) est éduquée pour se raccourcir en adoptant un profil courbe ou ondulé dès lors que la température dépasse le seuil austénitique, et chacune des branches latérales (2, 3, 4) se recourbe en direction du centre de l'agrafe à une telle température.

Dans une autre forme de réalisation de l'invention représentée sur les figures 17 et 18, l'agrafe comporte également trois branches latérales (2, 3, 4), mais selon une forme en tabouret. Les trois branches sont alors chacune constituée par repliage partiel sur lui-même du fil constitutif de l'agrafe, et sont éduquées pour se recourber en direction du centre de l'agrafe à une température supérieure au seuil austénitique du matériau. Parallèlement, à une telle température, les portions de raccordement entre lesdites branches sont éduquées pour adopter un profil cintré, de telle sorte à réduire la distance séparant lesdites branches latérales. De fait, ce type d'agrafe est d'application pour la réduction des fractures osseuses multiples, du type métacarpien.

Dans le même esprit, on a représenté sur les figures 19, 20 et 21 une agrafe en forme de tabouret, mais comportant quatre branches latérales régulièrement réparties. Dans la réalisation décrite, chacune des branches est constituée par un repliage partiel du fil, les deux extrémités libres du fil se rejoignant par exemple au niveau de l'une des branches. Lors du passage au dessus du seuil austénitique Aₛ du matériau, les différentes portions de raccordement (10 - 13) subissent une modification de forme selon une forme ondulée ou cintrée, induisant un raccourcissement de chacune des diagonales constituant la base du tabouret.

Dans la forme de réalisation illustrée sur la figure 21, on peut prévoir que seules deux portions de raccordement opposées, par exemple (11, 12) soient éduquées de telle sorte à présenter une forme ondulée, et partant un raccourcissement de la distance séparant les deux couples de branches latérales (2, 4) et (3, 9) lors du passage de la température au dessus du seuil austénitique, et ce en fonction du choix du chirurgien.

On conçoit donc que de par l'adoption d'un fil unitaire monobloc pour la réalisation de ces agrafes multi-branches, de même que leur éducation en vue de leur conférer une mémoire de forme bien spécifique est beaucoup plus aisée. En outre, compte tenu du double effet de compression souhaitée, de leur capacité auto-rétentive et de la stabilité mécanique accrue qu'elles induisent au niveau du foyer de fracture, ces agrafes s'avèrent tout particulièrement adaptées pour la réduction de nombreux types de fractures.

## Revendications

1. Agrafe monobloc d'ostéosynthèse réalisée en un alliage martensitique thermo-élastique, dont les températures de transformation martensitique Mₛ et austénitique Aₛ peuvent varier, en fonction des applications, entre - 20 ° C à 70 ° C comportant des branches latérales (2, 3, 4, 9) destinées à être insérées de part et d'autre du foyer de la fracture d'un os à réparer, lesdites branches latérales étant raccordées entre elles par au moins une portion de raccordement (5, 6, 7, 8), les branches latérales et la portion de raccordement (5 - 8) étant éduquées pour respectivement se recourber sensiblement vers le centre de l'agrafe et pour se raccourcir sous l'effet de la température, lorsque celle-ci dépasse la température de transformation austénitique Aₛ dudit matériau, ***caractérisée*** en ce qu'elle est constituée d'un fil unitaire constitué par ledit alliage, avec lequel au moins l'une (2) des branches latérales (2, 3, 4, 9) qui la constituent est réalisée par repliage au moins partiel dudit fil sur lui-même.

2. Agrafe d'ostéosynthèse selon la revendication 1, ***caractérisée*** en ce que deux (3, 4) des branches latérales sont constituées par les deux extrémités du fil.

3. Agrafe d'ostéosynthèse selon la revendication 2, ***caractérisée*** en ce que toute branche latérale (9) supplémentaire est réalisée par repliage partiel du fil sur lui-même.

4. Agrafe d'ostéosynthèse selon l'une des revendications 1 à 3, ***caractérisée*** en ce qu'une partie de la portion de raccordement (7, 8) de l'agrafe, destinée à se raccourcir lors du passage à une température supérieure à la température de transformation austénitique du matériau martensitique qui la constitue, est constituée par repliage partiel sur lui-même du fil qui la constitue dans le prolongement de la branche latérale (2) ainsi constituée, le raccourcissement étant obtenu par adoption de ladite portion d'un profil courbe ou ondulé.

5. Agrafe d'ostéosynthèse selon l'une des revendications 1 à 3, ***caractérisée*** en ce que le raccourcissement de la portion de raccordement de l'agrafe à une température supérieure à la température de transformation austénitique du matériau martensitique qui la constitue, est obtenu par rabat partiel des prolongements unitaires (5, 6) de ladite portion en direction de la branche latérale (2, 9) constituée par repliage partiel sur lui-même du fil qui la constitue.

6. Agrafe d'ostéosynthèse selon l'une des revendications 4 et 5, ***caractérisée*** en ce que le raccourcissement de la portion de raccordement de l'agrafe à une température supérieure à la température de transformation austénitique du matériau martensitique qui la constitue, est obtenu par effet combiné de l'adoption d'un profil recourbé de la zone de ladite portion (7, 8) constituée par repliage partiel sur lui-même du fil constituant l'agrafe, et du rabat en direction de la branche latérale (2, 9) des zones unitaires (5, 6) de ladite agrafe.

7. Agrafe d'ostéosynthèse selon l'une des revendications 1 à 6, ***caractérisée*** en ce qu'elle comporte trois branches latérales (2, 3, 4), raccordées entre elles par une portion de raccordement (5 - 8) en forme de Y, les deux zones unitaires supérieures (5, 6) de ladite portion de raccordement ainsi que les branches latérales (3, 4) les prolongeant étant susceptibles de se rapprocher ou de s'écarter l'une de l'autre lors du passage à une température supérieure à la température de transformation austénitique du matériau composant le fil de l'agrafe.

8. Agrafe d'ostéosynthèse selon la revendication 7, ***caractérisée*** en ce que chaque brin de fil de la branche latérale (2) est en outre éduqué pour adopter un profil ondulé ou courbe lors du passage à une température supérieure à la température de transformation austénitique du matériau constitutif du fil.

9. Agrafe d'ostéosynthèse selon l'une des revendications 1 à 4, ***caractérisée*** en ce qu'elle comporte trois branches latérales (2, 3, 4), raccordées entre elles par une portion de raccordement (5 - 8) en forme de T, les deux branches latérales (3, 4) constituées par les extrémités libres du fil constituant l'agrafe, étant éduquées pour se recourber en direction l'une de l'autre à une température supérieure à la température de transformation austénitique du matériau qui le compose, et les zones unitaires supérieures (5, 6) ainsi que les branches latérales (3, 4) les prolongeant étant susceptibles de se rapprocher l'une de l'autre à une telle température.

10. Agrafe d'ostéosynthèse selon l'une des revendications 1 à 4, ***caractérisée*** en ce qu'elle comporte trois branches latérales (2, 3, 4), raccordées entre elles par une portion de raccordement (5 - 8) en forme de V, dans laquelle l'une (2) des branches latérales (2, 3, 4) est constituée par repliage partiel sur lui-même du fil constituant l'agrafe, et dans laquelle les deux autres branches latérales (3, 4) sont suscpetibles de se rapprocher ou de s'écarter l'une de l'autre lors du passage à une température supérieure à la température de transformation austénitique du matériau constitutif dudit fil.

11. Agrafe d'ostéosynthèse selon l'une des revendications 1 et 3, ***caractérisée*** en ce qu'elle comporte trois branches latérales (2, 3, 9), raccordées entre elles par une portion de raccordement propre à conférer à l'agrafe une forme en tabouret, dans laquelle chacune des branches latérales (2, 3, 9) est constituée par repliage partiel du fil constitutif de l'agrafe sur lui-même.

12. Agrafe d'ostéosynthèse selon l'une des revendications 1 à 6, ***caractérisée*** en ce qu'elle comporte comporte quatre branches latérales (2, 3, 4, 9), raccordées entre elles par une portion de raccordement (5 - 8) en forme de double Y montés bout à bout, deux (2, 9) des branches latérales étant constituées par repliage partiel sur lui-même du fil constitutif de l'agrafe, et les deux autres branches (3, 4) étant constituées par les deux extrémités libres dudit fil, les deux branches de chacun des deux jeux étant susceptibles de se rapprocher ou de s'écarter, en fonction de l'éducation en mémoire de forme qui leur est donnée, lors du passage de la température à une température supérieure à la température de transformation austénitique du matériau constitutif du fil.

13. Agrafe d'ostéosynthèse selon l'une des revendications 1 à 4, ***caractérisée*** en ce qu'elle comporte quatre branches latérales (2, 3, 4, 9), raccordées entre elles par une portion de raccordement en forme de double T, deux (2, 9) des branches latérales étant réalisées par repliage partiel sur lui-même du fil constitutif de l'agrafe, les deux autres (3, 4) étant constituées par les deux extrémités libres dudit fil.

14. Agrafe d'ostéosynthèse selon la revendication 13, ***caractérisée*** en ce que les branches latérales (2, 3, 4, 9) sont éduquées de telle sorte à présenter un profil légèrement cintrée à une température inférieure à la température de transformation martensitique du matériau composant l'agrafe, et un profil nettement plus recourbé à une température supérieure à la température de transformation austénitique dudit matériau, le cintrage s'effectuant sensiblement dans le plan intégrant les deux jeux respectifs (2, 9) et (3, 4) de branches latérales.

15. Agrafe d'ostéosynthèse selon l'une des revendications 1 à 4, ***caractérisée*** en ce qu'elle comporte quatre branches latérales (2, 3, 4, 9), reliées entre elles par des portions de raccordement (10 - 13) conférant à l'agrafe une forme en tabouret, chacune des branches latérales constituant l'un des pieds dudit tabouret étant constituée par le repliage partiel sur lui-même du fil constitutif de l'agrafe, les deux extrémités libres du fil venant se rejoindre au niveau de l'une des branches, au moins deux portions de raccordement non consécutives étant éduquées pour adopter un profil ondulé ou courbe induisant un raccourcissement des portions concernées.

## Claims

1. A monobloc osteosynthesis clip made of a thermoelastic martensitic alloy, the martensitic and austenitic transformation temperatures Mₛ and Aₛ of which can vary, depending on the applications, between -20°C to 70°C, including side branches (2,3,4,9) intended to be inserted on either side of the focus of the fracture of a bone to be repaired, said side branches being connected together by at least one connection portion (5, 6, 7, 8), the side branches and the connection portion being educated in order respectively to bend substantially towards the center of the clip and to shorten under the effect of temperature when it exceeds the austenitic transformation temperature Aₛ of said material, wherein it consists of a unitary wire consisting of said alloy, with which at least one (2) of the side branches (2, 3, 4, 9) which constitute it is made by at least partial folding back of said wire on itself.

2. The osteosynthesis clip as claimed in claim 1,wherein two (3, 4) of the side branches consist of the two ends of the wire.

3. The osteosynthesis clip as claimed in claim 2, wherein any additional side branch (9) is made by partial folding back of the wire on itself.

4. The osteosynthesis clip as claimed in one of claims 1 to 3, wherein a part of the connection portion (7, 8) of the clip, intended to shorten when passing to a temperature greater than the austenitic transformation temperature of the martensitic material which constitutes it, consists of partial folding back on itself of the wire which constitutes it in the extension of the side branch (2) thus constituted, the shortening being obtained by adoption by said portion of a curved or corrugated profile.

5. The osteosynthesis clip as claimed in one of claims 1 to 3, wherein the shortening of the connection portion of the clip at a temperature greater than the austenitic transformation temperature of the martensitic material which constitutes it is obtained by partial folding down of the unitary extensions (5, 6) of said portion in the direction of the side branch (2, 9) consisting of the partial folding back on itself of the wire which constitutes it.

6. The osteosynthesis clip as claimed in one of claims 4 and 5, wherein the shortening of the connection portion of the clip at a temperature greater than the austenitic transformation temperature of the martensitic material which constitutes it is obtained by combined effect of the adoption of a curved profile by the zone of said portion (7,8) consisting of partial folding back on itself of the wire constituting the clip and of the folding down in the direction of the side branch (2,9) of the unitary zones (5,6) of said clip.

7. The osteosynthesis clip as claimed in one of claims 1 to 6, wherein it includes three side branches (2,3,4), connected together by a Y-shaped connection portion (5-8), the two upper unitary zones (5,6) of said connection portion as well as the side branches (3,4) extending them being capable of moving together or apart with respect to one another when passing to a temperature greater than the austenitic transformation temperature of the material forming the wire of the clip.

8. The osteosynthesis clip as claimed in claim 7,wherein each strand of wire of the side branch (2) is furthermore educated to adopt a corrugated or curved profile when passing to a temperature greater than the austenitic transformation temperature of the constituent material of the wire.

9. The osteosynthesis clip as claimed in one of claims 1 to 4, wherein it includes three side branches (2,3,4) connected together by a T-shaped connection portion (5-8), the two side branches (3,4) consisting of the free ends of the wire constituting the clip being educated to curve toward one another at a temperature greater than the austenitic transformation temperature of the material which forms it, and the upper unitary zones (5,6) as well as the side branches (3,4) extending them being capable of moving toward one another at such a temperature.

10. The osteosynthesis clip as claimed in one of claims 1 to 4, wherein it includes three side branches (2,3,4) connected together by a V-shaped connection portion (5-8), in which one (2) of the side branches (2,3,4) consists of partial folding back on itself of the wire constituting the clip, and in which the other two side branches (3,4) are capable of moving together or apart with respect to one another when passing to a temperature greater than the austenitic transformation temperature of the constituent material of said wire.

11. The osteosynthesis clip as claimed in one of claims 1 and 3, wherein it includes three side branches (2,3,9) connected together by a connection portion capable of giving the clip a stool shape, in which each of the side branches (2,3,9) consists of partial folding back of the constituent wire of the clip on itself.

12. The osteosynthesis clip as claimed in one of claims 1 to 6, wherein it includes four side branches (2,3,4,9) connected together by a connection portion in double-Y shape (5-8), mounted end-to-end, two (2,9) of the side branches consisting of partial folding back on itself of the constituent wire of the clip, and the other (3,4) two branches consisting of the two free ends of said wire, the two branches of each of the two sets being capable of moving together or apart, depending on the shape-memory education given to them, when the temperature passes to a temperature greater than the austenitic transformation temperature of the constituent material of the wire.

13. The osteosynthesis clip as claimed in one of claims 1 to 4, wherein it includes four side branches (2,3,4,9) connected together by a connection portion in double-T shape, two (2,9) of the side branches being made by partial folding back on itself of the constituent wire of the clip, the other two (3,4) consisting of the two free ends of said wire.

14. The osteosynthesis clip as claimed in claim 13, wherein the side branches (2,3,4,9) are educated so as to have a slightly bent profile at a temperature lower than the martensitic transformation temperature of the material forming the clip, and a markedly more curved profile at a temperature greater than the austenitic transformation temperature of said material, the bending taking place substantially in the plane incorporating the two respective sets (2,9) and (3,4) of side branches.

15. The osteosynthesis clip as claimed in one of claims 1 to 4, wherein it includes four side branches (2,3,4,9) connected together by connection portions (10-13) giving the clip a stool shape, each of the side branches constituting one of the feet of said stool consisting of the partial folding back on itself of the constituent wire of the clip, the two free ends of the wire joining at one of the branches, at least two non-consecutive connection portions being educated to adopt a corrugated or curved profile leading to a shortening of the portions in question.

## Patentansprüche

1. Osteosyntheseklammer, die aus einem Stück besteht, hergestellt aus einer thermoelastischen martensitischen Legierung, deren martensitische Umwandlungstemperatur Mₛ und deren austenitische Umwandlungstemperatur Aₛ in Abhängigkeit der Anwendungen von -20 °C bis 70 °C variieren kann, mit seitlichen Beinen (2, 3, 4, 9), die dazu vorgesehen sind, beidseits des Fokus einer zu behebenden Knochenfraktur eingetrieben zu werden, wobei die seitlichen Beine über zumindest einen Verbindungsabschnitt (5, 6, 7, 8) untereinander verbunden sind, wobei die seitlichen Beine und der Verbindungsabschnitt einen solchen Memory-Effekt aufweisen, daß, falls die austenitische Umwandlungstemperatur Aₛ des genannten Materials überschritten wird, unter Temperatureinwirkung, diese sich etwas auf das Zentrum der Klammer zu krümmen und verkürzen, dadurch gekennzeichnet, daß die Klammer aus einem Einzeldraht besteht, der aus der genannten Legierung gebildet ist, aus dem zumindest eines (2) der diese aufbauenden seitlichen Beine (2, 3, 4, 9) zumindest teilweise durch Umbiegen in bifilarer Art hergestellt ist.

2. Osteosyntheseklammer nach Anspruch 1, dadurch gekennzeichnet, daß zwei (3, 4) der seitlichen Beine durch die beiden äußeren Enden des Drahtes gebildet sind.

3. Osteosyntheseklammer nach Anspruch 2, dadurch gekennzeichnet, daß jedes weitere seitliche Bein (9) durch teilweises Umbiegen in bifilarer Art hergestellt ist.

4. Osteosyntheseklammer nach einem der Ansprüche 1 bis 3, dadurch gekennzeichnet, daß ein Teil des Verbindungsabschnittes (7, 8) der Klammer, der dazu bestimmt ist, bei Überschreiten einer Temperatur über der austenitischen Umwandlungstemperatur des diese aufbauenden martensitischen Materials sich zu verkürzen, durch teilweises Umbiegen in bifilarer Art des Drahtes ausgebildet ist, der die Verlängerung des so gebildeten seitlichen Beines (2) ist, wobei die Verkürzung dadurch erreicht wird, daß diesem Abschnitt ein gekrümmtes oder gewelltes Profil verliehen wird.

5. Osteosyntheseklammer nach einem der Ansprüche 1 bis 3, dadurch gekennzeichnet, daß die Verkürzung des Verbindungsabschnittes der Klammer bei einer Temperatur über der austenitischen Umwandlungstemperatur des diese aufbauenden martensitischen Materials durch teilweises Biegen der einzeldrahtigen (5, 6) Verlängerungen dieses Abschnittes in Richtung desjenigen seitlichen Beines (2, 9) erhalten wird, das durch Umbiegen des diese aufbauenden Drahtes in bifilarer Art hergestellt wurde.

6. Osteosyntheseklammer nach einem der Ansprüche 4 oder 5, dadurch gekennzeichnet, daß die Verkürzung des Verbindungsabschnittes der Klammer bei einer Temperatur über der austenitischen Umandlungstemperatur des diese aufbauenden martensitischen Materials durch die Kombination der Wirkungen des Vorsehens eines gekrümmten Profiles des Bereichs des genannten Abschnittes (7, 8) der durch teilweises Umbiegen des die Klammer aufbauenden Drahtes in bifilarer Art gebildet ist und durch Biegen in Richtung der seitlichen Beine (2, 9) der einzeldrahtigen Bereiche (5, 6) der genannten Klammer erhalten wird

7. Osteosyntheseklammer nach einem der Ansprüche 1 bis 6, dadurch gekennzeichnet, daß sie drei seitliche Beine (2, 3, 4) aufweist, die über einen Verbindungsabschnitt (5-8) in Form eines Y untereinander verbunden sind, wobei die beiden oben liegenden einzeldrahtigen Bereiche (5, 6) des genannten Verbindungsabschnittes sowie die seitlichen Beine (3, 4), die diese verlängern, in der Lage sind, bei Überschreiten einer Temperatur, die höher liegt als die austenitische Umwandlungstemperatur des den Draht der Klammer bildenden Materials sich einander anzunähern oder voneinander weg zu bewegen.

8. Osteosyntheseklammer nach Anspruch 7, dadurch gekennzeichnet, daß jeder Strang des Drahtes des seitlichen Beines (2) darüber hinaus einen solchen Memory-Effekt aufweist, daß er ein gewelltes oder gekrümmtes Profil einnimmt, wenn auf eine Temperatur übergegangen wird, die über der austenitischen Umwandlungstemperatur des den Draht bildenden Materials liegt.

9. Osteosyntheseklammer nach einem der Ansprüche 1 bis 4, dadurch gekennzeichnet, daß sie drei seitliche Beine (2, 3, 4) aufweist, die über einen Verbindungsabschnitt (5-8) in Form eines T untereinander verbunden sind, wobei diejenigen beiden seitlichen Beine (3, 4), die durch die freien Enden des die Klammer ausbildenden Drahtes gebildet sind, mit einem solchen Memory-Effekt versehen sind, daß sie sich bei einer Temperatur, die über der austenitischen Umwandlungstemperatur des diese aufbauenden Materials auf einander zu krümmen, und daß die darüber liegenden einzeldrahtigen Bereiche (5, 6) sowie die seitlichen Beine (3, 4), die diese verlängern, in der Lage sind, sich bei einer solchen Temperatur gegenseitig anzunähern.

10. Osteosyntheseklammer nach einem der Ansprüche 1 bis 4, dadurch gekennzeichnet, daß sie drei seitliche Beine (2, 3, 4) aufweist, die über einen Verbindungsabschnitt (5-8) in Form eines V untereinander verbunden sind, wobei eines (2) der seitlichen Beine (2, 3, 4) durch zumindest teilweises Umbiegen in bifilarer Art des die Klammer aufbauenden Drahtes ausgebildet ist, und bei der die beiden anderen seitlichen Beine (3, 4) in der Lage sind, wenn eine Temperatur erreicht wird, die über der austenitischen Umwandlungstemperatur des den Draht aufbauenden Materials liegt, sich aufeinander zu oder voneinander weg zu bewegen.

11. Osteosyntheseklammer nach einem der Ansprüche 1 oder 3, dadurch gekennzeichnet, daß sie drei seitliche Beine (2, 3, 9) aufweist, die über einen Verbindungsabschnitt untereinander verbunden sind, der so ausgebildet ist, daß dieser der Klammer die Form eines Hockers verleiht, wobei jedes der seitlichen Beine (2, 3, 9) durch teilweises Umbiegen des die Klammer aufbauenden Drahtes in bifilarer Art ausgebildet ist.

12. Osteosyntheseklammer nach einem der Ansprüche 1 bis 6, dadurch gekennzeichnet, daß sie vier seitliche Beine (2, 3, 4, 9) aufweist, die über einen Verbindungsabschnitt (5-8) in Form eines am unteren Ende zusammengefügten doppelten Y aufweist, wobei zwei (2, 9) seitliche Beine durch teilweises Umbiegen in bifilarer Art des die Klammer aufbauenden Drahtes ausgebildet sind, und die anderen beiden Beine (3, 4) durch die beiden freien Enden des genannten Drahtes ausgebildet sind, wobei die beiden Beine jedes der beiden Sätze in der Lage sind, sich aufeinander zu zu bewegen oder voneinander weg zu bewegen, und zwar in Abhängigkeit des ihnen verliehenen Formengedächtnisses, und zwar bei Erreichen einer Temperatur, die über der austenitischen Umwandlungstemperatur des den Draht aufbauenden Materiales liegt.

13. Osteosyntheseklammer nach einem der Ansprüche 1 bis 4, dadurch gekennzeichnet, daß sie vier seitliche Beine (2, 3, 4, 9) aufweist, die untereinander über einen Verbindungsabschnitt in Form eines doppelten T verbunden sind, wobei zwei (2, 9) der seitlichen Beine durch teilweises Umbiegen des die Klammer aufbauenden Drahtes in bifilarer Art gebildet sind, und die beiden anderen (3, 4) durch die beiden freien Enden des Drahtes gebildet sind.

14. Osteosyntheseklammer nach Anspruch 13, dadurch gekennzeichnet, daß die seitlichen Beine (2, 3, 4) einen solchen Memory-Effekt aufweisen, daß sie bei einer Temperatur unterhalb der martensitischen Umwandlungstemperatur des die Klammer aufbauenden Materials ein etwas gewölbtes Profil aufweisen und bei einer Temperatur über der austenitischen Umwandlungstemperatur des genannten Materials ein wesentlich stärker gekrümmtes Profil aufweisen, wobei sich die Wölbung merklich in der Ebene abspielt, in der sich die jeweiligen Sätze (2, 9 und 3, 4) der seitlichen Beine befinden.

15. Osteosyntheseklammer nach einem der Ansprüche 1 bis 4, dadurch gekennzeichnet, daß sie vier seitliche Beine (2, 3, 4, 9) aufweist, die über Verbindungsabschnitte (10-13) untereinander verbunden sind, die der Klammer die Form eines Hockers verleihen, wobei jedes der seitlichen Beine einen Fuß des Hockers bildet, die durch teilweises Umbiegen des die Klammer aufbauenden Drahtes in bifilarer Art ausgebildet sind, wobei die beiden freien Enden des Drahtes auf Höhe eines der Beine zusammengefügt sind, wobei zumindest zwei nicht aufeinander folgende Verbindungsabschnitte mit einem solchen Memory-Effekt versehen sind, daß ein gewelltes oder gekrümmtes Profil eingenommen wird, das ein Verkürzen der betreffenden Abschnitte verursacht.
